# EUROPEAN PATENT SPECIFICATION

(11) **EP 2 893 071 B1**
(45) Date of publication and mention of the grant of the patent: **16.12.2020**
(21) Application number: 13773446.3
(22) Date of filing: 10.09.2013
(51) Int. Cl.: D04H 13/00, A61F 13/49, B32B 5/02, B32B 5/08, B32B 5/26, B32B 7/14, B32B 3/08, B29C 48/00

(54) **METHODS FOR FORMING AN ELASTIC STRIP LAMINATE**
VERFAHREN ZUR HERSTELLUNG EINES ELASTISCHEN STREIFENVERBUNDSTOFF
PROCÉDÉ DE FABRICATION D' UNE BANDE ÉLASTIQUE LAMINÉE

(30) Priority: 10.09.2012 US 201261699242 P
(43) Date of publication of application: 15.07.2015
(73) Proprietor: Tredegar Film Products LLC, North Chesterfield, VA 23225 (US)
(72) Inventor: BRUCE, Stephen, D., Montpelier, VA 23192 (US)
(74) Representative: den Braber, Gérard Paul
(86) International application number: PCT/US2013/059001
(87) International publication number: WO 2014/040048

(56) References cited:
- US-A- 5 733 628
- US-A1- 2002 086 602
- US-A1- 2005 287 892
- US-A1- 2006 292 328
- US-A1- 2009 047 855
- US-B1- 6 447 875

## Description

### BACKGROUND

An absorbent device such as a disposable diaper typically includes a topsheet, an absorbent core, and a backsheet. The topsheet contacts skin of the user of the device and may transmit fluid exudate toward the absorbent core such that the absorbent core may captivate and store the fluid exudate. The backsheet serves as the outer most layer and provides a liquid barrier against staining or soiling clothing near the device by preventing leakage or the passing through of liquids stored in the core. Typically, the topsheet, absorbent core, and backsheet are attached to, sealed to, adhered to, bonded to, and/or molded to each other.

In most absorbent devices, an elastic ear is also attached to, sealed to, adhered to, bonded to, and/or molded to, for example, the backsheet or topsheet, or both. Such an elastic ear can be stretched and attached to a portion of the backsheet to form the sides and leg openings of the absorbent article, to provide a comfortable fit for different anatomies of various users of the absorbent device, to reduce leakage, and the like. The elastic ear typically includes an elastic laminate with an elastic material and one or more nonwoven materials. Unfortunately, current elastic laminates used for such elastic ears tend to be expensive in both material costs and processing costs. For example, an elastic material currently is applied continuously in a cross-direction (CD) and machine direction (MD) of the elastic laminate, and, as such, a larger amount of elastic material is used thereby increasing cost of the elastic laminates.

To reduce the amount of elastic material, individual strips of elastic materials may be initially cut and subsequently attached to one or more of the nonwoven materials of the elastic laminate. Unfortunately, to currently manufacture elastic laminates with individual strips of elastic materials, additional and/or separate processing is often required, thus adding to the manufacturing cost of this laminate. In addition, the lamination equipment is often specialized requiring such equipment as web guiding devices, hold-down belts, and special adhesive application equipment, thereby increasing processing complexity and cost as well as the time to manufacture (e.g. the number of processes or steps used to manufacture the elastic laminate). Additionally, the handling of such individual elastic strips after manufacture, but before being attached to one or more of the nonwoven webs typically tends to be tedious and problematic.

Another manufacturing difficultly using current techniques or processes may also include adjusting for "growth" where the dimensions of a laminate change dimensions when subjected to processing conditions such as changes in the basis weight of nonwoven materials, activation settings, and the like. Unfortunately, today, dimensional tolerances normally need to be reversed engineered to take into account such growth and to achieve the desired end dimensions of the laminate. Such reverse engineering may also increase processing complexity and the manufacturing cost.

### SUMMARY

A method is disclosed according to claim 1, for forming a laminate with elastic materials extruded thereon, the method comprising:receiving a first nonwoven web; extruding an elastic material onto the first nonwoven web such that the elastic material is directly applied to the first nonwoven web continuously in a machine direction and discontinuously in a cross direction to form an elastic laminate with one or more elastic strips on the first nonwoven web; and activating via stretching the elastic laminate only at the location of the one or more elastic strips, along the entire length of each of the elastic strips. Additionally, in an embodiment, the elastic material may be applied unevenly or non-uniformly in the cross direction when forming the elastic strips on the first nonwoven web during extrusion such that more elastic material may be on the edges of an elastic strip than in the middle portion of an elastic strip and, thus, an elastic strip may have a non-uniform thickness across its width in the cross-direction. Additionally, the elastic material may be applied in the cross-direction such that the elastic strips may be non-uniformly spaced across the width of the first nonwoven web or material. As described herein, the elastic laminate may reduce material and manufacturing costs as well as improve the manufacturing method or process thereof.

In one embodiment, a second nonwoven web may be bonded to the elastic laminate (e.g. the first nonwoven web with the one or more elastic strips). Additionally, the elastic laminate including the first nonwoven web with the one or more elastic strips with or without the second nonwoven bonded thereto may be activated. After activation, the first nonwoven web with the one or more elastic strips with or without the second nonwoven bonded thereto of the elastic laminate may be the same size (e.g. width) as before activation (e.g. the original sizes such as width). In embodiments, the elastic laminate may be cut into a pattern or a portion of the elastic laminate may be cut into a pattern and/or the portion of the elastic laminate may be assembled in an article such as an absorbent article.

The Summary is provided to introduce a selection of concepts in a simplified form that are further described below in the Detailed Description.

### BRIEF DESCRIPTION OF THE DRAWINGS

A more detailed understanding of the embodiments disclosed herein may be had from the following description, given by way of example in conjunction with the accompanying drawings.
FIG. 1A depicts an example embodiment of a process or method for forming an elastic laminate with an elastic strip extruded onto a nonwoven.
FIG. 1B depicts an example embodiment of an extrusion that may be provided and/or used in the process or method of FIG. 1A.
FIG. 1C, 1D, and 1E depict example embodiments of an activation that may be provided and/or used in the process or method of FIGs. 1A and/or 1B.
FIG. 2 depicts an example embodiment of a portion of an elastic laminate that includes an elastic strip extruded on a nonwoven.
FIGs. 3A and 3B depict example embodiments of a perspective view and an end view, respectively, of a portion of the elastic laminate of FIG. 2.
FIG. 4 depicts an example embodiment of a perspective view of a portion of the elastic laminate of FIGs. 2 and 3A-3B with another layer of a nonwoven adhered thereto.
FIG. 5 depicts an example embodiment of a perspective view of a portion of the elastic laminate of FIGs. 2 and 3A-3B with another layer of a nonwoven adhered thereto after activation.
FIG. 6 depicts an example embodiment of a portion of the elastic laminate of FIG. 2 cut along lines A-A, B-B, and C-C that may be used in an absorbent article.
FIG. 7 depicts an example embodiment of an absorbent article with a portion of the elastic laminate used therein.

### DETAILED DESCRIPTION

A detailed description of illustrative embodiments will now be described with reference to the various Figures.

Systems, methods, or processes may be disclosed for forming an elastic laminate that may be used as an elastic ear for an absorbent article such as a diaper, an adult incontinence implement, and the like. According to an embodiment, the elastic laminate may include a nonwoven web and at least one elastic strip or lane that may be smaller than the nonwoven web (e.g. the width of the nonwoven web). For example, the elastic strip or lane may be continuous in one direction such as a machine direction, rather than being continuous across the cross-direction and machine-direction of the nonwoven web, to reduce the amount of elastic material used and, thus, the cost of the elastic laminate. To form the elastic laminate, the elastic strip or lane may be extruded directly onto the nonwoven web such that the elastic material associated therewith may not need to be cut into a strip and then separately adhered or attached to the nonwoven web as done in typical or current methods or processes, thus, further reducing the cost of the elastic laminate incurred during processing (e.g. equipment cost and/or processing time). Additionally, such an extrusion of the elastic strip or lane directly on the nonwoven web may further improve the manufacturing of the elastic laminate by eliminating the handling of the elastic strips after being cut and before being attached or adhered to the nonwoven web. This improvement may be particularly important when a large number of elastic strips, for example 4 to 40 or more, may be cut and handled separately prior to attaching to one or more nonwoven webs. The process or method described herein may, thus, be capable of manufacturing elastic laminates of one to three or more meters in width and may reduce manufacturing complexity and cost.

According to an example embodiment, by selecting a draw gap (e.g. a distance from the die to the cast roller), a "neck-in" of the elastic material may be controlled in such a way as to create heavy areas or edges (e.g. a non-uniform thickness across width of the strips) on the strips that may act as "tack-down" zones (e.g. as described herein). These heavy areas on the elastic strips may further be created by other means such as by designing the die with a variation in the gap at the opening of the die lip, and the like. Such zones may help reduce creep in the laminate instead of maintaining its integrity in the stretched state. A laminate may experience creep when the elastic strip delaminates from the nonwoven when the laminate may be stretched for an extended period and, the elastic strip may prematurely recover or retract during use, losing its integrity and causing an absorbent article to lose contact with a user.

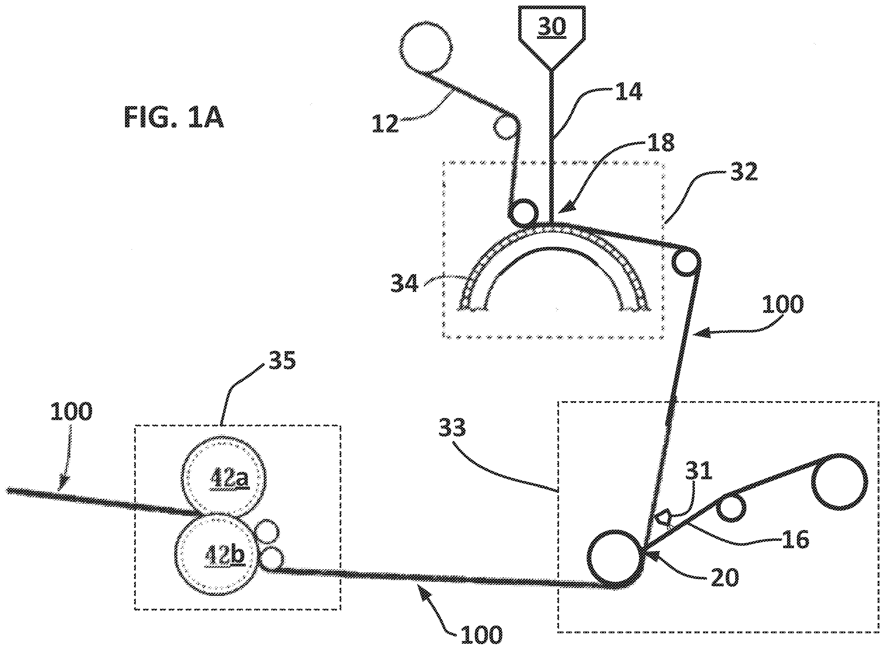

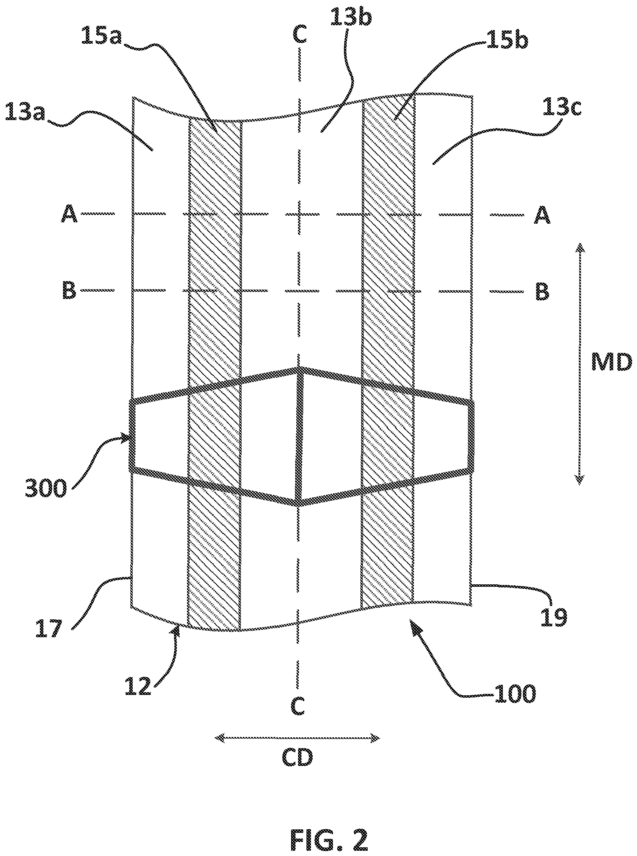

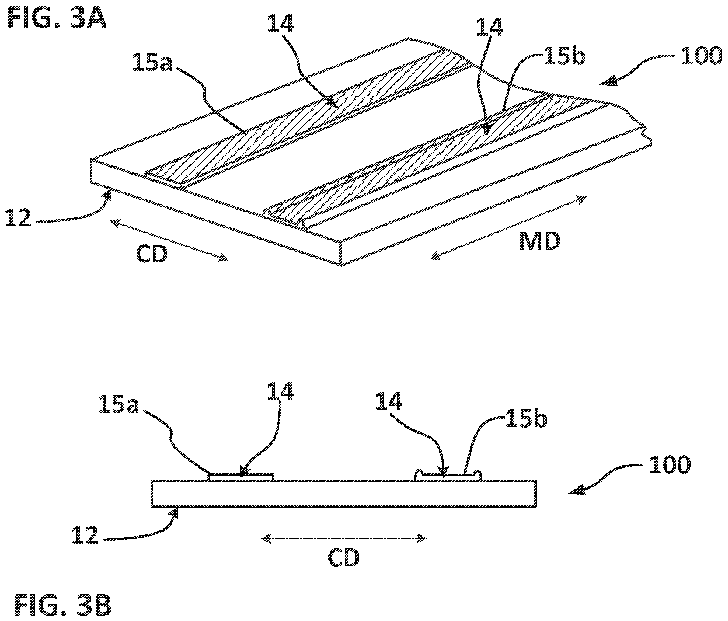

FIGs. 1A-1E depict example embodiments of processes or methods or portions of a process or method for forming an elastic laminate with an elastic strip extruded onto a nonwoven. For example, as shown in FIG. 1A, an elastic material 14 may be received from a die 30 at an extrusion stage 32 shown in FIG. 1A where the elastic material 14 may be directly extruded onto a nonwoven web or material 12 such as a first nonwoven web or material (e.g. as shown in FIG. IB). According to an example embodiment, the extrusion stage 32 may be a vacuum lamination process or an extrusion lamination process that may be used to produce a portion of an elastic laminate such as an elastic laminate 100 shown in FIGs. 2 and 3A-3B. For example, the nonwoven web or material 12 may be received by and/or placed into contact with a roller 34 such as a vacuum lamination drum (e.g. the roller 34 may include a screen that may rotate around a vacuum slot (not shown)) or any other suitable roller or drum. In example embodiments using vacuum lamination as described herein, the elastic material 14 may be apertured or un-apertured during the extrusion stage 32 or other stages of the process shown in FIG. 1A depending on the process conditions. For example, an extrusion vacuum lamination or coating process (e.g. at 32) may or may not impart apertures or a three-dimensional shape to the elastic material 14 or nonwoven web or material 12. U.S. Pat. No. 5,733,628, describes an example embodiment for imparting apertures or three-dimensional shape during the vacuum extrusion lamination or coating process. When the extrusion vacuum lamination or coating process (e.g. at 32) may not impart apertures or a three-dimensional shape to the elastic material 14 or nonwoven web or material 12, additional forming and/or aperturing processes or methods for creating breathability (e.g. using pin perforation or other suitable equipment that may deform or pierce the elastic material 14 or nonwoven web or material 12 or using) (not shown) known to the industry may be performed to impart holes.

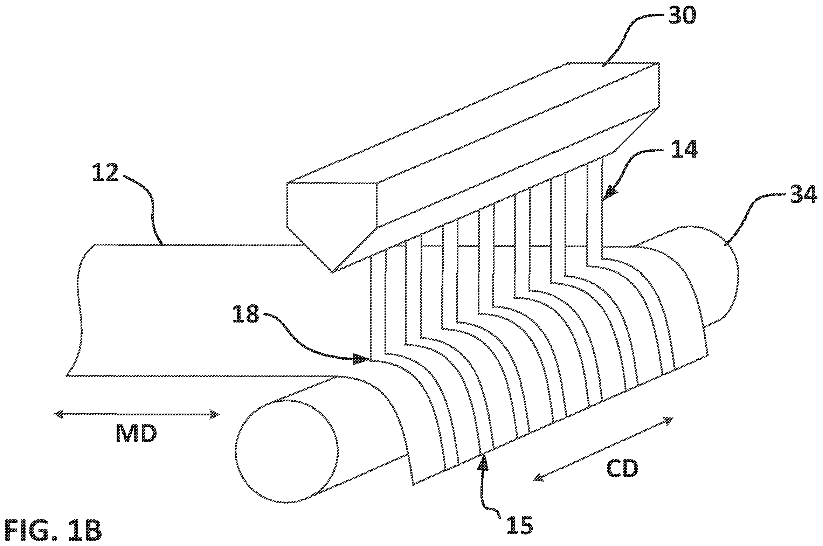

The elastic material 14 in a molten or semi-molten state is extruded in elastic strips or lanes directly onto the nonwoven web 12 in contact with the roller 34. As shown in FIGs. 1A and 1B, the elastic material 14 in the molten or semi-molten state is provided from the die 30 positioned above the roller 34 such that the elastic material 14 may be directly applied to the nonwoven web or material 12 (e.g. at an extrusion point 18) continuously in one direction such as the machine-direction (MD) and discontinuously in other directions such as the cross-direction (CD) in one or more elastic strips or lanes 15 (i.e. extruded onto the nonwoven web or material 12) as shown in FIGs. 2 and 3A-3B.

In an alternate embodiment (not shown), at the extrusion stage 32 (via an extrusion or vacuum lamination process), the elastic material 14 may be extruded from the die 30 onto the roller 34 in one or more strips or lanes and then the nonwoven web or material 12 may be brought in contact with the elastic material 14 such that the elastic material 14 may be between the roller 34 and the nonwoven web or material 12.

Additionally, as described herein, the elastic material 14 in the molten or semi-molten state may be extruded from the die 30 such that the elastic strips or lanes 15 of elastic material 14 may not be applied uniformly spaced in the cross-direction (CD) or may the elastic material 14 may be of different thickness across the width of the elastic strips or lanes 15 in the CD (e.g. to help reduce creep) when extruding the elastic strips or lanes 15 on the nonwoven web 12.

Furthermore, in such an extrusion (e.g. at 32), the elastic material 14 in the molten or semi-molten state may bond with the nonwoven web or material 12 as the elastic material 14 cools (e.g. after the extrusion point 18 where the elastic material 12 may be applied to the nonwoven web or material 12).

Additionally, in embodiments, the elastic material 14 may be extruded onto the nonwoven web 12 into the one or more elastic strips or lanes 15 using other suitable lamination or extrusion techniques (e.g. along with or alternatively to the process shown in FIG. 1A) to form an elastic laminate 100 (e.g. as further shown in FIGs. 2 and 3A-3B) with one or more elastic strips or lanes 15 on the nonwoven web or material 12.

As described above, FIGs. 2 and 3A-3B depict example embodiments of at least a portion of an elastic laminate that includes at least one elastic strip or lane extruded on a nonwoven web at, for example, the extrusion stage 32 of the method or process described in FIG. 1A. As shown in FIGs. 2 and 3A-3B, the elastic laminate 100 includes may the nonwoven web 12 with one or more elastic strips or lanes 15a, 15b extruded directly on a surface of the nonwoven web or material 12 as described above such that the elastic material 14 associated with the elastic strips or lanes 15a, 15b is continuous in one direction such as the machine-direction (MD) and is discontinuous in other directions such as the cross-direction (CD).

Additionally, as described above, the elastic material 14 that may be used for the elastic strips 15a, 15b may or may not be uniform across the cross-direction (CD) of the elastic strips 15a, 15b. For example, as described above, more elastic material 14 may be applied when forming the outer edges of the elastic strip 15b than the middle portion of the elastic strip 15b during extrusion to help reduce creep that may arise as described herein. Also, the elastic material 14 may be applied with a uniform thickness across the cross-direction (CD) of the elastic strip 15a.

As shown in FIGs. 2 and 3A-3B, after extruding the elastic strips 15a, 15b on the nonwoven web or material 12, nonwoven portions 13a, 13b, and 13c may be disposed on opposite sides of the elastic strips 15a, 15b. According to an example embodiment, the nonwoven portions 13a, 13b, and 13c may be located along edges 17 and 19 respectively of the elastic laminate 100, may be adjacent to, and may separate or be separated by the elastic strips 15a, 15b.

Additionally, the elastic laminate 100 with the nonwoven portions 13a, 13b, and 13c and elastic strips or lanes 15a, 15b extruded directly thereon, for example, via vacuum lamination as shown in FIG. 1A or another suitable extrusion lamination process or method may be a bilaminate elastic laminate produced thereby. According to an example embodiment, the bilaminate elastic laminate may include an adhesive such that the elastic laminate 100 may further be adhesive laminated to a second nonwoven web 16 as described herein to create a trilaminate elastic laminate.

As shown, in an embodiment, the elastic laminate 100 may enable a simplified manufacturing process or method as described above with respect to FIG. 1A and 1B as the elastic strips or lanes (e.g. 15 or 15a, 15b) may not be handled separately (e.g. after being cut) as such elastic strips or lanes may be extruded directly onto the nonwoven.

In an example embodiment, the elastic laminate 100 along with the nonwoven web or material 12 and/or 16 described herein may measure about 110 mm to about 190 mm wide in the cross-direction (CD), and in an embodiment may be 150 mm wide in the CD. Additionally, each of the elastic strips or lanes 15a, 15b may measure about 20 mm to about 130 mm wide in the CD, and in example embodiments may be about 35 mm to about 70 mm wide or about 35 mm to about 45 mm wide in the CD. Furthermore, the nonwoven portions 13a, 13b, and 13c, in total, may measure the width of the elastic laminate 100 or the first or second nonwoven web or material 12 or 16 less the width of the elastic strips or lanes 15a, 15b. The width of the nonwoven portions 13a and 13c located along edges 17 and 19 may further match or be equal to the other as shown in FIG. 2 and 3A-3B.

According to example embodiments, the elastic material 14 and the elastic strips or lanes 15 or 15a, 15b formed therefrom may include a styrene-block copolymer such as styrene/isoprene/styrene (SIS), styrene/butadiene/styrene (SBS), or styrene/ethylene-butadiene/styrene (SEBS), styrene/ethylene-propylene/styrene block copolymers, , and the like; blends of different styrene block copolymers; blends of stryrene block copolymers with polyolefins such as polyethylene (PE), polypropylene (PP), polystyrene (PS), and the like; polyolefin based elastomers such as Dow®Infuse™, ExxonMobile® Vistamaxx®, and the like; and/or any combination thereof or any other suitable elastic material. In embodiments, a monolayer elastic strip or lane may be used. It should be understood, however, that an elastic strip or lane having multiple layers may also be used. For example, an elastic core may be provided or used between two skin layers to enhance bonding to the nonwoven layers or to facilitate processability. Suitable skin layers are well known and may include, for example, polyethylene which may be more or less elastic than the elastic material. The thickness of the elastic strip or lane may vary, although the individual layers of the films may be typically thin (e.g., the elastic core is usually, but not necessarily, less than 100 microns, and skin layers, if used, are usually less than 20 microns).

In one embodiment, the elastic material 14 and/or the elastic strips or lanes 15 or 15a, 15b formed therefrom may include a blend of SIS, polyethylene, and polystyrene either as a monolayer or a coextruded with skin layers. Additionally, as described herein, the elastic material 14 and the elastic strips or lanes 15 or 15a, 15b formed therefrom may also be a monolayer film, may be a coextruded film, and the like in embodiments.

Additionally, in embodiments, the nonwoven web or materials 12 and/or 16 may be a fibrous material or web such as staple fiber materials including thermal bonded carded fibers, air through bonded carded fibers, spunlace fibers, spunbond or continuous fibers, and the like that may be made of polyethylene (PE), polypropylene (PP), a bicomponent or blends of PE and PP, or other materials. For example, suitable nonwovens may include loose fibers and webs prepared using know techniques such as, for example, air laying, spunbond, spun lace, bonded melt blown, thermobond, bonded carded. Additionally, the nonwoven material may be homogeneous or contain a variety of woven materials including bi-component fibers (e.g. having an inner core of one material and an outer core of a second material), fibers of different morphologies, geometries, and surface finishes. Suitable nonwovens materials may also include, for example, fibrous polyolefins such as polyethylenes and polypropylenes, and natural fibers such as cotton and cellulose.

According to an example embodiment, the properties and/or materials of the elastic material 14 and/or the nonwoven materials or webs 12 and/or 16 may be selected and/or manipulated to meet one or more benchmarks that may be desired for the elastic laminate, and, thus, the elastic ear that may be formed therefrom. For example, in an embodiment, the properties and/or materials of the elastic material 14 and/or the nonwoven materials or webs 12 and/or 16 may be selected and/or manipulated such that a piece of elastic laminate cut in the shape of 300 in Figure 2, with a dimension of 50mm along edge 17 and 100mm along line C-C when gripped in the nonwoven lanes 13a and 13b and subjected to a 10N stretching force in the cross-direction may stretch about 10mm to about 70 mm in one embodiment, about 20 mm to about 60 mm in an additional embodiment, and about 30 to about 50 mm in yet another embodiment.

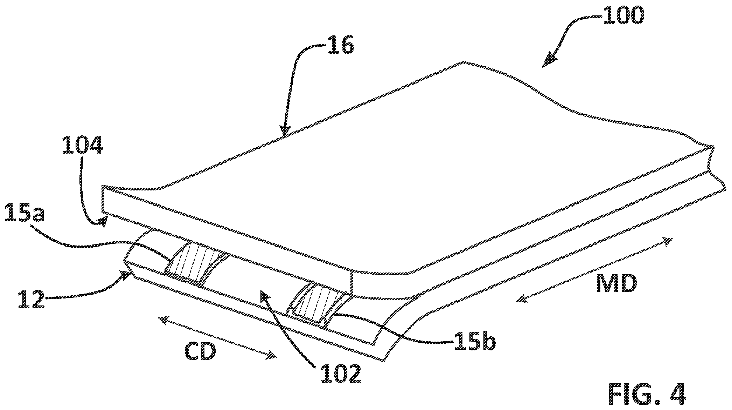

Referring back to FIGs. 1A and 4, according to an additional embodiment, another nonwoven web or material 16 such as a second nonwoven web or material may be introduced and bonded to the nonwoven web or material 12 with the elastic material 14 extruded thereon in one or more elastic strips or lanes 15 at a bonding stage 33. For example, the roller 34 that may have the nonwoven web 12 in contact therewith may rotate such that the elastic material 14 may be extruded directly on the nonwoven web or material 12 in a continuous direction into the one or more elastic strips or lanes (e.g. 15 or 15a, 15b) as described above. After extrusion, the nonwoven web or material 12 with the one or more elastic strips or lanes (e.g. 15 or 15a, 15b) of elastic material 14 may be subsequently passed to a bonding point 20. Prior to the bonding point 20, the other nonwoven web or material 16 may be passed under an adhesive applicator 31. At bonding point 20 the nonwoven web 12 with one or more elastic strips (e.g. 15 or 15a or 15b) and the second nonwoven web 16 are passed through a nip (not shown) to bond the nonwoven web 12 and second nonwoven web 16 together. In another embodiment, the other nonwoven web or material 16 may be bonded to the surface of the nonwoven web or material 12 (e.g. with the elastic strips or lanes of elastic material 14) by a point bonding process or method such as thermal or ultrasonic bonding or other suitable processes or methods.

As described above, in an alternate embodiment (not shown) at the extrusion stage 32 (via an extrusion or vacuum lamination process), the elastic material 14 may be extruded from the die 30 onto the roller 34 in one or more strips or lanes and then the nonwoven web or material 12 may be brought in contact with the elastic material 14 such that the elastic material 14 may be between the roller 34 and the nonwoven web or material 12 to extrude the one or more elastic strips thereon. In such an embodiment, the nonwoven web or material 16 may be bonded to the opposite side of the laminate in the extrusion process 32 such that the nonwoven web or material 16 may be bonded to the surface of the first nonwoven 12 with the one or more elastic strips or lanes 15 or 15a, 15b.

FIG. 4 depicts an example embodiment of a perspective view of a portion of the elastic laminate of FIGs. 2 and 3A-3B with another layer of a nonwoven web adhered thereto. For example, in one embodiment, the elastic laminate 100 may further include another nonwoven web or material 16 (e.g. a second nonwoven material web) that may be bonded to the nonwoven web or material 12 with the elastic strips or lanes 15a, 15b associated with the elastic material 14. As shown in FIG. 4, a first surface 102 of the nonwoven web or material 12 with the elastic strips or lanes 15a, 15b may be bonded to a first surface 104 of the other nonwoven web 16. According to an example embodiment, the nonwoven web or material 12 with the elastic strips or lanes 15a, 15b may be bonded to the other nonwoven web 16 by adhering the fibers associated with the nonwoven material web 12 to the fibers associated with the other nonwoven web 102 using adhesive, thermal or ultrasonic bonding or other suitable processes or methods.

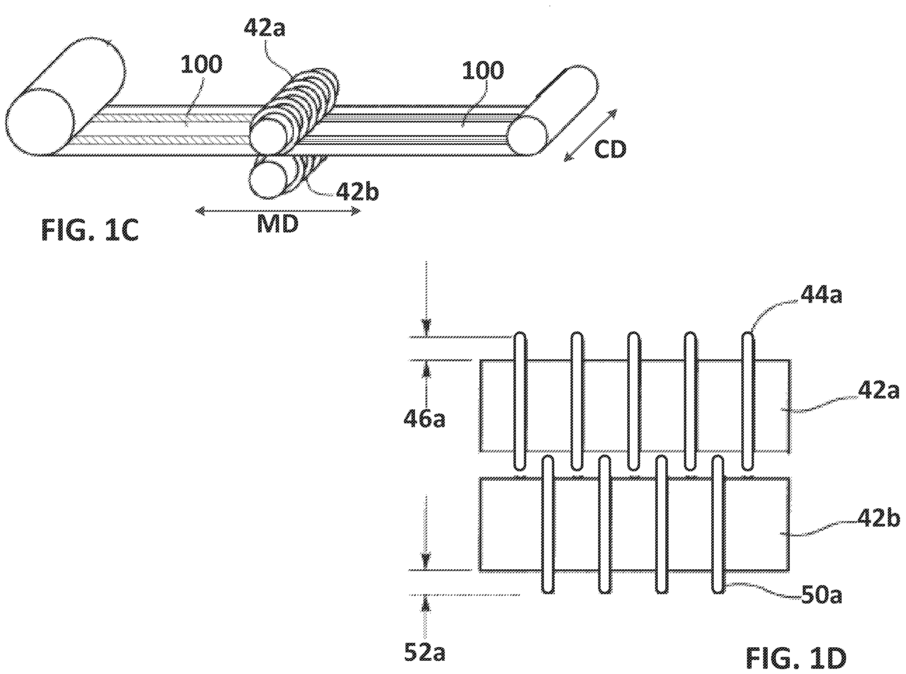

As shown in FIG. 1A and 1C-1E, after the nonwoven web or material 12 may be extruded with the one or more strips or lanes of elastic material 14 at the extrusion stage 32 and/or may be bonded to the second non-elastic material 16 at the bonding stage 33, the elastic laminate (e.g. 100) that may be formed therefrom may be further stretched or activated at an activation stage 35. As shown in FIG. 1A, the elastic laminate (e.g. 100 that may include the nonwoven web or material 12 with the one or more strips or lanes of elastic material 14 or elastic strips or lanes 15 or 15a, 15b) may be activated or stretched using, for example, parallel activation rollers 42a, 42b such as heated, un-heated, or cooled intermeshing gear (IMG) rollers. Additionally, in embodiments, the nonwoven web or material 12 and/or 16 may be pre-activated (e.g. before the extrusion stage 32, bonding stages 33, and/or activation stage 35) to further improve stretch and/or softness.

In an embodiment, the parallel activation rollers 42a, 42b may have circumferentially oriented teeth (e.g. as shown in FIGs. 1C-1E) that may intermesh and thereby stretch the elastic laminate in a direction as it passes between the parallel activation rollers 42a, 42b. For example, the elastic laminate may be passed through the parallel activation rollers 42a, 42b in a machine direction. The parallel activation rollers 42a, 42b with the teeth thereof may then stretch the elastic laminate in the cross direction and may break the nonwoven web or material 12 and/or 16 in a direction that may be substantially perpendicular to the machine direction, i.e., the cross direction. Alternatively, the activation rollers 42a, 42b and the teeth thereof may stretch the elastic laminate and may break the nonwoven web or material 12 and/or 16 in the machine direction. In another embodiment, the elastic laminate can be subjected to multiple activations (e.g. two activation stages including one that stretches in the machine direction and one that stretches in the cross direction).

For example, for stretching the elastic laminate in the cross-direction, as shown in FIGs. 1D and IE, the activation roller 42a may include circumferentially spaced teeth 44a, 44b. The teeth 44a shown in FIG. ID may have an engagement height 46a and may be equally spaced along the circumference of the activation roller 42a. Similarly, the activation roller 42b shown in FIG. ID that may be parallel and opposite of the activation roller 42a may include teeth 50a. The teeth 50a may have an engagement height 52a and may be equally spaced along the circumference of the activation roller 42b. As shown in FIG. IE, the teeth 44b may have an engagement height 46b. Additionally, the teeth 44b may be grouped into lanes or activation lanes with spaces in between each of the teeth 44b. In an example embodiment, each of the activation lanes may also include a space 47b therebetween. The activation roller 42b shown in FIG. IE that may be parallel and opposite of the activation roller 42a may include teeth 50b along the entire length such that the roller 42b may be fully toothed. Alternately, the roller 42b may also have teeth grouped into lanes or activation lanes, such that the activation lanes in roller 42b are aligned to engage with the activation lanes in roller 42a. According to example embodiments, the teeth 44a, 44b, 50a, and/or 50b may be about 2.54 mm (0.100") to about 12.7 mm (0.500") deep, about 0.254mm (0.010") to about 7.62 mm (0.300") wide, and may be spaced apart about 0.254mm (0.010") to about 7.62 mm (0.300").

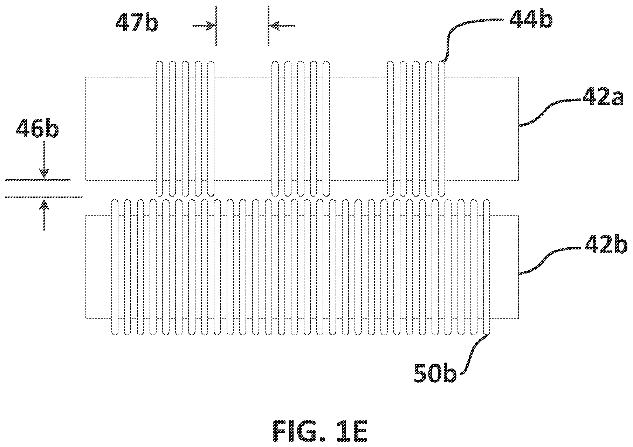

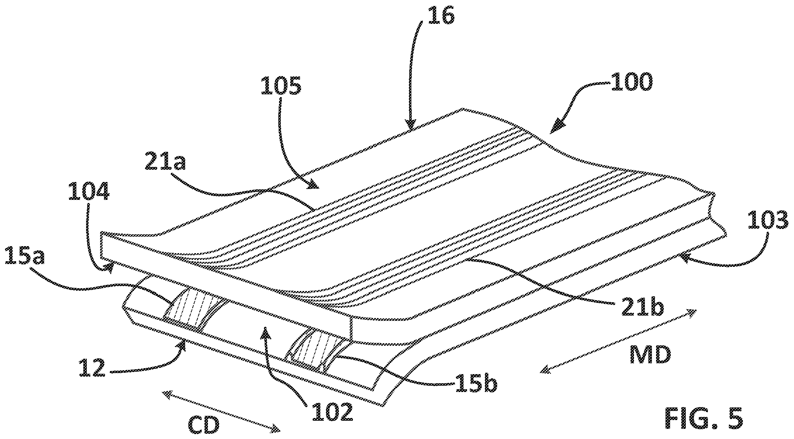

As shown in FIGs. 1C-1E, the teeth 44a, 44b of the activation roller 42a and the teeth 50a, 50b of the activation roller 42b may be spaced along the respective circumferences of the activation rollers 42a, 42b respectively such that the elastic laminate 100 may pass through or between one or more of the respective teeth 44a, 50a, 44band/or 50b. In an example embodiment, as the elastic laminate 100 (e.g. the nonwoven web or material 12 with the strips or lanes of elastic or elastic material 14 with (trilaminate) or without (bilaminate) the nonwoven web or material 16 further bonded thereto) may travel through and between the teeth 44a, 50a, 44b and/or 50b, respectively, the elastic laminate may be activated (e.g. represented in FIG. 5 as 21a and 21b). While traveling through or between the respective teeth 44a, 50a, 44b and/or, 50b, the elastic laminate may be subjected to stretching such that the portions of the nonwoven web or materials 12, 16 in contact with the elastic strips or lanes 15a, 15b) may be aligned with the tangential intersection of the teeth 44b and 50b in Figure IE and, as such, those portions of the elastic laminate may be elastically overstrained during activation. The elastic overstraining may occur by applying a relatively high strain rate and an extension ratio of at least 200 percent while adjacent regions located between the strips (the portions of the nonwoven web or materials 12, 16 in space 47b in Figure 1E) may be strained from 0 percent to 200 percent. Such an effect may activate the activated strips (e.g. 21a and 21b of FIG. 5) of the elastic laminate 100 and the nonwoven materials and webs 12 and 16 to behave more like the elastic material 14 (e.g. may be easier to stretch). As such, in an example embodiment, one or more portions of the elastic laminate (e.g. that may include elastic strips of elastic material) may be stretched independently to create local zones of elastically recoverable material.

The dimensions of a laminate may change during processing and manufacturing, such as during an activation stage (e.g., stage 35) and is referred to as "growth". As such, dimensional tolerances normally need to be reversed engineered from what results from the processing or manufacturing such that the desired end dimensions of the laminate may be achieved. For example, the basis weight of the nonwovens and the setting for activation such as the depth of teeth of the rollers used during activation (e.g. the rollers 42a, 42b such as the activation rollers) to give more or less stretch of the laminate may affect the growth associated with the laminate. However, during processing or manufacturing using the processes or methods in FIG. 1A and the materials described herein, such as during activation at the activation stage 35, the elastic laminate 100 may not have a significant growth. For example, recovery after activating the elastic laminate 100 during the activation stage 35 may be close to 100% such that the elastic laminate 100 including the nonwoven web or material 12 and/or 16, the elastic strips or lanes 15 or 15a, 15b, and the like to account for growth after activation may be about the same size as prior to activation or another processing or manufacturing method shown in FIG. 1A (e.g. may be about the same size as an original size of the nonwoven web or material 12 and/or 16, the extruded elastic strips or lanes 15 or 15a, 15b, and the like prior to activation). As such, the equipment such as the rollers 42a, 42b in FIG. 1A and/or the processing or manufacturing settings performed by the equipment in FIG. 1A may not need to be reversed engineered based on different material basis weights, differences in activation roller settings, and the like thereby enabling greater manufacturing flexibility. Additionally, as the growth of the elastic laminate 100 may be negligible when undergoing activation at the activation stage 35 in FIG. 1A, the activation performed at the activation stage 35 on the elastic laminate 100 may be increased thereby enabling the elastic laminate 100 to be even easier to stretch or have greater stretching properties.

FIG. 5 depicts an example embodiment of a perspective view of a portion of the elastic laminate of FIG. 4 after activation (e.g. 35). For example, the elastic laminate 100 may further include an activated strip 21a, 21b that may be observed on a second surface 105 nonwoven web 16 and on nonwoven web 12 (not shown) where elastic laminate 100 may be subjected to stretching and elastically overstrained. As such, while traveling through the respective teeth (e.g. 44a, 50a, 44b and/or 50b) of the rollers (e.g. 42a and 42b) that may be used during activation, the elastic strips or lanes 15a, 15b may be stretched and elastically overstrained such that the stretching and/or overstraining may produce or impart or produce the activation strips 21a, 21b on the second surface 105 of the nonwoven web 16 and nonwoven web 12 opposite of the first surface 104 bonded to the nonwoven web or material 12 with the elastic strips or lanes 15a, 15b extruded thereon. As shown in FIG. 5, the activation strips 21a, 21b may approximately correspond to the position or location of the elastic strips or lanes 15a, 15b. Corresponding activated strips (not shown) may also be produce or imparted onto a second surface 103 of the first nonwoven web or material 12 opposite to the first surface 102 on which the elastic strips or webs 15a, 15b may be extruded. Additionally, as shown in FIG. 5, the elastic laminate 100 may not have a significant growth. For example, the elastic laminate 100 including the nonwoven web or material 12 and/or 16, the elastic strips or lanes 15 or 15a, 15b, and the like shown in FIG. 5 after activation may be about the same size as the elastic laminate 100 shown in FIG. 4 prior to activation. Additionally, the activation strips or lanes (e.g. 21a, 21b) may encompass the full width of the elastic strips or lanes (e.g. 15 or 15a, 15b) or only a portion across the width of such elastic strips or lanes (e.g. in the cross direction). For example, when the nonwoven portions not in contact with the elastic strips or lanes are activated, a weak area may be created in the laminate causing premature breakage in use. As such, in an embodiment, about 2 to about 10 mm from the edge of the elastic strips or lanes (e.g. 15 or 15a, 15b) is activated such that the activation is not in the nonwoven portions (e.g. 13a, 13b, or 13c) and is not in contact with the elastic strips or lanes.

In an example embodiment, the elastic laminate 100 after activation (e.g. at 35) may be easier to stretch that the elastic laminate 100 prior to activation. For example, the elastic laminate 100 after activation with the activation strips 21a, 21b may have a lower modulus (e.g. may stretch easier and more) than the nonwoven web or material 12, 16 and elastic laminate before activation Additionally, in embodiments, the elastic laminate 100 after activation may have a 5% tensile force on a one inch wide test strip that is 2-10 times greater in the unactivated lanes (between the elastic strips) than in the activated lanes 21a and 21b. Tensile force is tested via ASTM D882 (2 inch draw gap, 20 inches/min, 1 inch wide specimen width).

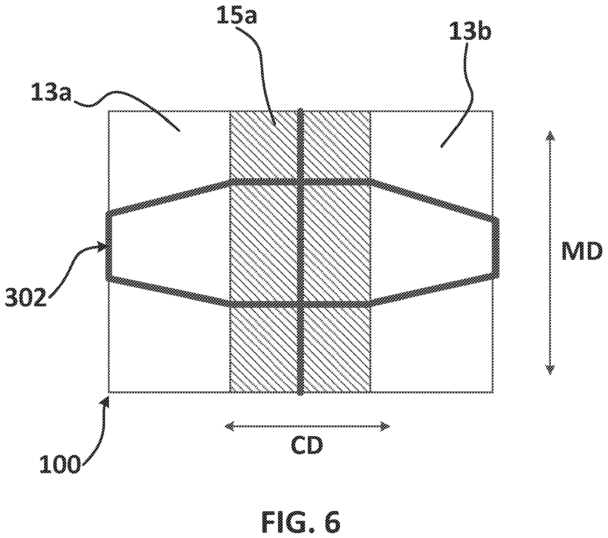

After activation, the elastic laminate 100 may then be passed on to other manufacturing processes or methods and/or collected onto a film roll for further processing at a subsequent time. For example, the elastic laminate 100 may be cut into various sizes or portions that may be used in an elastic ear that may be included in an absorbent article. As shown in FIG. 2, the elastic laminate 100 may be cut (e.g. die cut) into a pattern 300 that may be used, for example, for the elastic ear. Additionally, FIG. 6 depicts an example embodiment of a portion of the elastic laminate of FIG. 2 along lines A-A, B-B, and C-C that may be cut (e.g. die cut) into a pattern 302 that may be used, for example, for the elastic ear.

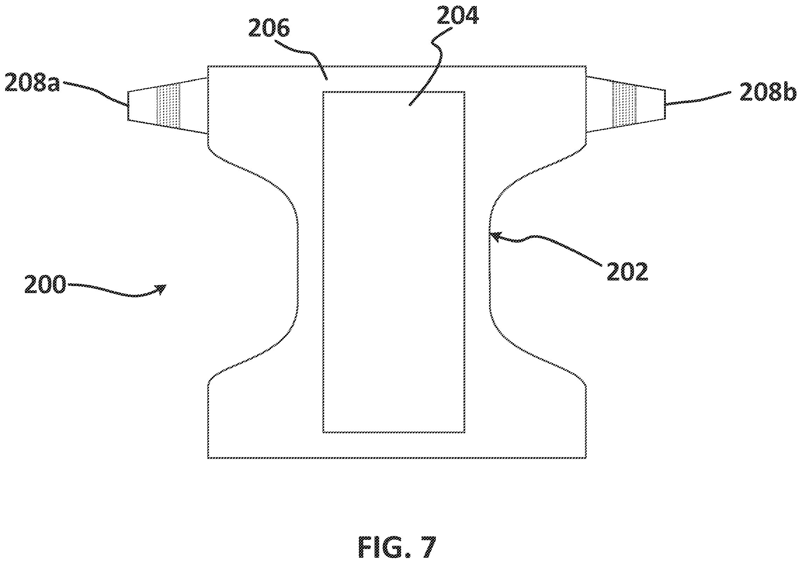

Additionally, the elastic laminate 100 or the cut portions thereof (e.g. in a pattern 300 and/or pattern 302) may be processed such that the elastic laminate 100 or the cut portions thereof (e.g. in pattern 300 and/or pattern 302) may be assembled into an absorbent article. FIG. 7 depicts an example embodiment of an absorbent article with a portion of an elastic laminate being used as an elastic ear of the absorbent article. As shown in FIG. 7, an absorbent article 200 such as a disposable diaper, adult incontinence implement or article, and the like may include a fluid collection portion 202 that may include a topsheet 204, a backsheet 206, and/or an absorbent core (not shown) between the topsheet 202 and the backsheet 206. The topsheet 202 may contact skin of the user of the article 200 and may transmit fluid exudate toward the absorbent core such that the absorbent core may captivate and store the fluid exudate. The backsheet 206 may be adjacent to the absorbent core opposite of the topsheet 202, may serve as the outer most layer, and/or may provide a liquid barrier against staining or soiling clothing near the article by preventing leakage or the passing through of liquids stored in the absorbent core. Typically, the topsheet 202, absorbent core, and backsheet 206 may be attached to, sealed to, adhered to, bonded to, and/or molded to each other.

Additionally, an elastic ear 208a and/or 208b may also be attached to, sealed to, adhered to, bonded to, and/or molded to, for example, the backsheet 206, the topsheet, or both. The elastic ear 208a and/or 208b may be stretched to form the sides and leg openings of the absorbent article, to provide a comfortable fit for different anatomies of various users of the absorbent article 200, to reduce leakage, and the like. The elastic ear 208a and/or 208b may further include a fastener (not shown) to keep the absorbent article 200 secured to the user thereof. In an example embodiment, the elastic ear 208a and/or 208b may include the cut portion of the elastic laminate 100 described herein.

## Claims

1. A method for forming a laminate with elastic materials extruded thereon, the method comprising:
receiving a first nonwoven web (12);
extruding an elastic material (14) onto the first nonwoven web (12) such that the elastic material (14) is directly applied to the first nonwoven web (12) continuously in a machine direction and discontinuously in a cross direction to form an elastic laminate (100) with one or more elastic strips (15) on the first nonwoven web (12); and
activating via stretching the elastic laminate (14) only at the location of the one or more elastic strips (15), along the entire length of each of the elastic strips (15).

2. The method of claim 1, wherein the elastic material is applied non-uniformly in the cross direction when extruding the elastic strips on the first nonwoven web such that at least one of the elastic strips have a non-uniform thickness across the width thereof.

3. The method of claim 1, wherein the elastic material is at least molten or semi-molten, and bonds to the first nonwoven web after an extrusion point when the elastic material cools.

4. The method of claim 1, wherein the elastic material is extruded onto the first nonwoven web via vacuum lamination.

5. The method of claim 1, further comprising:
bonding a second nonwoven web to the first nonwoven web with the one or more elastic strips extruded thereon associated with the elastic laminate prior to activating.

6. The method of claim 5, wherein the second nonwoven web is bonded to the first nonwoven web with the one or more elastic strips extruded thereon via adhesive, thermal or ultrasonic bonding.

7. The method of claim 5, further comprising:
cutting a portion of the elastic laminate including the first nonwoven web with the one or more elastic strips and the second nonwoven bonded thereto into a pattern after activation.

8. The method of claim 7, further comprising:
assembling the portion of the elastic laminate into an absorbent article.

9. An elastic laminate comprising:
a first nonwoven web (12); and
one or more elastic strips (15) of an elastic material extruded directly onto a first surface of the first nonwoven web (12), wherein the one or more elastic strips (15) extruded directly onto the first surface of the first nonwoven web (12) are continuous in a machine direction and discontinuous in a cross direction,
wherein the elastic laminate (100) is activated via stretching only at the location of the one or more elastics strips (15), along the entire length of each of the elastic strips (15).

10. The elastic laminate of claim 9, wherein a thickness of the elastic material of the one or more elastic strips is not uniform across the width of the one or more elastic strips in the cross direction of the one or more elastic strips.

11. The elastic laminate of claim 9, wherein the elastic material of the one or more elastic strips comprises at least one of the following: a styrene-block copolymer, a stryrene-block copolymer with polyolefins, a polyolefin based elastomer, or blends thereof.

12. The elastic laminate of claim 9, wherein the elastic material of the one or more elastic strips comprises an elastic core and non-elastic skin layers.

## Patentansprüche

1. Verfahren zum Herstellen eines Laminats mit darauf extrudierten elastischen Materialien, wobei das Verfahren Folgendes umfasst:
Aufnehmen eines ersten Vliesnetzes (12);
Extrudieren eines elastischen Materials (14) auf das erste Vliesnetz (12), sodass das elastische Material (14) kontinuierlich in einer Maschinenrichtung und diskontinuierlich in einer Querrichtung direkt auf das erste Vliesnetz (12) aufgetragen wird, um ein elastisches Laminat (100) mit einem oder mehreren elastischen Streifen (15) auf dem ersten Vliesnetz (12) zu bilden; und
Aktivieren durch Dehnen des elastischen Laminats (14) nur an der Stelle von einem oder mehreren elastischen Streifen (15), entlang der gesamten Länge jedes der elastischen Streifen (15).

2. Verfahren nach Anspruch 1, wobei das elastische Material ungleichmäßig in der Querrichtung aufgetragen wird, wenn die elastischen Streifen auf das erste Vliesnetz extrudiert werden, sodass wenigstens einer der elastischen Streifen eine ungleichmäßige Dicke entlang der Breite davon aufweist.

3. Verfahren nach Anspruch 1, wobei das elastische Material wenigstens geschmolzen oder halbgeschmolzen ist und sich nach einem Extrusionspunkt an das erste Vliesnetz bindet, wenn das elastische Material abkühlt.

4. Verfahren nach Anspruch 1, wobei das elastische Material mittels Vakuumlaminierung auf das erste Vliesnetz aufgetragen wird.

5. Verfahren nach Anspruch 1, weiter Folgendes umfassend:
Binden eines zweiten Vliesnetzes an das erste Vliesnetz mit dem einen oder mehreren darauf extrudierten elastischen Streifen, assoziiert mit dem elastischen Laminat, vor dem Aktivieren.

6. Verfahren nach Anspruch 5, wobei das zweite Vliesnetz mit dem einen oder mehreren darauf extrudierten elastischen Streifen über Adhäsiv-, Thermo- oder Ultraschallbindung an das erste Vliesnetz gebunden ist.

7. Verfahren nach Anspruch 5, weiter Folgendes umfassend:
Schneiden eines Abschnitts des elastischen Materials, das erste Vliesnetz enthaltend, mit dem einen oder mehreren elastischen Streifen und das zweite daran gebundene Vlies in ein Muster nach der Aktivierung.

8. Verfahren nach Anspruch 7, weiter Folgendes umfassend:
Zusammenfügen des Abschnitts des elastischen Laminats in einen absorbierenden Artikel.

9. Elastisches Laminat, Folgendes umfassend:
ein erstes Vliesnetz (12); und
einen oder mehrere elastische Streifen (15) eines elastischen Materials, direkt extrudiert auf eine erste Oberfläche des ersten Vliesnetzes (12), wobei ein oder mehrere elastische Streifen (15), direkt auf eine erste Oberfläche des ersten Vliesnetzes (12) extrudiert, kontinuierlich in einer Maschinenrichtung und diskontinuierlich in einer Querrichtung sind,
wobei das elastische Laminat (100) entlang der gesamten Länge jedes der elastischen Streifen (15) nur an der Stelle von einem oder mehreren Elastik-Streifen (15) durch Dehnen aktiviert wird.

10. Elastisches Laminat nach Anspruch 9, wobei eine Dicke des elastischen Materials des einen oder mehrerer elastischer Streifen nicht gleichmäßig über die Breite des einen oder mehrerer elastischer Streifen in der Querrichtung des einen oder mehrerer elastischer Streifen ist.

11. Elastisches Laminat nach Anspruch 9, wobei das elastische Material des einen oder mehrerer elastischer Streifen wenigstens eines der Folgenden umfasst: ein Styren-Block-Copolymer, ein Styren-Block-Copolymer mit Polyolefinen, ein Polyolefinbasiertes Elastomer oder Mischungen davon.

12. Elastisches Laminat nach Anspruch 9, wobei das elastische Material des einen oder mehrerer elastischer Streifen einen elastischen Kern und nicht-elastische Hautschichten umfasst.

## Revendications

1. Procédé de formation d'un stratifié avec des matériaux élastiques extrudés sur celui-ci, le procédé comprenant :
la réception d'une première bande non tissée (12) ;
l'extrusion d'un matériau élastique (14) sur la première bande non tissée (12) de sorte que le matériau élastique (14) soit appliqué directement à la première bande non tissée (12) de manière continue dans un sens machine et de manière discontinue dans un sens transversal pour former un stratifié élastique (100) avec un ou plusieurs rubans élastiques (15) sur la première bande non tissée (12) ; et
l'activation via un étirage du stratifié élastique (14) uniquement à l'emplacement des un ou plusieurs rubans élastiques (15), le long de toute la longueur de chacun des rubans élastiques (15).

2. Procédé selon la revendication 1, dans lequel le matériau élastique est appliqué de manière non uniforme dans le sens transversal lors de l'extrusion des rubans élastiques sur la première bande non tissée de sorte qu'au moins un des rubans élastiques ait une épaisseur non uniforme transversalement à sa largeur.

3. Procédé selon la revendication 1, dans lequel le matériau élastique est au moins fondu ou semi-fondu, et se lie à la première bande non tissée après un point d'extrusion lorsque le matériau élastique refroidit.

4. Procédé selon la revendication 1, dans lequel le matériau élastique est extrudé sur la première bande non tissée via une stratification sous vide.

5. Procédé selon la revendication 1, comprenant en outre :
le liage d'une seconde bande non tissée à la première bande non tissée avec les un ou plusieurs rubans élastiques extrudés sur celle-ci associés au stratifié élastique avant activation.

6. Procédé selon la revendication 5, dans lequel la seconde bande non tissée est liée à la première bande non tissée avec les un ou plusieurs rubans élastiques extrudés sur celle-ci via un liage par adhésif, thermique ou par ultrasons.

7. Procédé selon la revendication 5, comprenant en outre :
le découpage d'une portion du stratifié élastique incluant la première bande non tissée avec les un ou plusieurs rubans élastiques et le second non tissé lié à celui-ci en un motif après activation.

8. Procédé selon la revendication 7, comprenant en outre :
l'assemblage de la portion du stratifié élastique en un article absorbant.

9. Stratifié élastique comprenant :
une première bande non tissée (12) ; et
un ou plusieurs rubans élastiques (15) d'un matériau élastique extrudé directement sur une première surface de la première bande non tissée (12), dans lequel les un ou plusieurs rubans élastiques (15) extrudés directement sur la première surface de la première bande non tissée (12) sont continus dans un sens machine et discontinus dans un sens transversal,
dans lequel le stratifié élastique (100) est activé via un étirage uniquement à l'emplacement des un ou plusieurs rubans élastiques (15), le long de toute la longueur de chacun des rubans élastiques (15).

10. Stratifié élastique selon la revendication 9, dans lequel une épaisseur du matériau élastique des un ou plusieurs rubans élastiques n'est pas uniforme transversalement à la largeur des un ou plusieurs rubans élastiques dans le sens transversal des un ou plusieurs rubans élastiques.

11. Stratifié élastique selon la revendication 9, dans lequel le matériau élastique des un ou plusieurs rubans élastiques comprend au moins l'un des suivants : un copolymère séquencé de styrène, un copolymère séquencé de styrène avec des polyoléfines, un élastomère à base de polyoléfine, ou des mélanges de ceux-ci.

12. Stratifié élastique selon la revendication 9, dans lequel le matériau élastique des un ou plusieurs rubans élastiques comprend une âme élastique et des couches de peau non élastiques.
